# EUROPEAN PATENT SPECIFICATION

(11) **EP 1 171 100 B1**
(45) Date of publication and mention of the grant of the patent: **30.07.2003**
(21) Application number: 00922609.3
(22) Date of filing: 05.04.2000
(51) Int. Cl.: A61K 9/08, A61K 31/59, A61P 5/00

(54) **SOLUBILIZED PHARMACEUTICAL COMPOSITION FOR PARENTERAL ADMINISTRATION**
GELÖSTE PHARMAZEUTISCHE ZUSAMMENSETZUNG FÜR DIE PARENTERALE VERABREICHUNG
COMPOSITION PHARMACEUTIQUE SOLUBILISEE A ADMINISTRATION PARENTERALE

(30) Priority: 13.04.1999 DK 48699
(43) Date of publication of application: 16.01.2002
(73) Proprietor: Leo Pharma A/S, 2750 Ballerup (DK)
(72) Inventor: DIDRIKSEN, Erik, Johannes, DK-2750 Ballerup (DK); GERLACH, Anette, Leth, DK-3450 Allerod (DK)
(74) Representative: Thalsoe-Madsen, Kine Birgit
(86) International application number: EP0003022
(87) International publication number: WO00061112

(56) References cited:
- EP-A- 0 800 825
- WO-A-96/36340
- WO-A-98/30205
- GB-A- 899 656
- GB-A- 1 123 433

## Description

The present invention concerns aqueous pharmaceutical compositions for parenteral use which contain a therapeutically and/or prophylactically active substance which has a low aqueous solubility. Furthermore, the active substance Is susceptible to degradation in an aqueous acidic environment.

### Background of the invention

An important route for administration of many drug substances is the parenteral route. Firstly, many drug substances are not sufficiently absorbed through the gastrointestinal mucosa to give a sufficient systemic concentration to obtain the desired therapeutic and/or prophylactic effect. Secondly, a relatively fast onset of a therapeutic effect is required in many cases and then parenteral administration of the drug substance in question is first choice, especially intravenous administration.

Compositions for parenteral use may be presented in the form of aqueous or oily solutions, dispersions, emulsions, suspensions, etc. Especially when a composition is to be administered Intravenously, it is preferred that the composition is in the form of a solution. However, a number of drug substances have a very low solubility in water, i.e. in order to obtain an aqueous composition which is in the form of a solution, it may be necessary to use a solvent mixture containing water and one or more organic solvents, such as a conventional co-solvent composition containing ethanol-propylene glycol.

Another possibility is to employ a solubilizer such as, e.g. Cremophor® EL. However, parenteral administration of compositions containing Cremophor® EL has previously been found to give rise to release of histamine (Lorenz, W. et al. in Agents and Actions, 1 982, **12 1/2,** 64-80 and Ennis, M. et al. in Agents and Actions, 1 985, 16 3/4, 265-268). The resulting anaphylactoid reactions in man following the administration of drug substances dissolved in Cremophor@ EL, pose a significant clinical problem. Eschalier, A. et al., Cancer Chemother Pharmacol 1988;21(3):246-50. have shown that Cremophor EL and Tween 80, which is another pharmaceutical excipient used as a solubilizer, induce non-specific histamine release. Therefore, attempts have been made to identify and/or develop solubilizers which have essentially no histamine release effect. Solutol® HS 15 manufactured by BASF, Germany, has been suggested as a solubilizer which is non-toxic and essentially without the unwanted histamine release effect, i.e. it is regarded as a low-histamine releaser.

Prior art compositions containing Solutol® HS 15 have a relatively high content of Solutol® HS 15 and, furthermore, other auxiliary components are included in the composition (e.g. emulsifiers, organic solvents, chelating agents, etc.). However, when developing pharmaceutical compositions - especially when developing compositions for parenteral use - it is an aim only to use non-toxic auxiliary substances which have a required function and only in the lowest possible amount to fulfil the required function. Therefore, there is still a need for developing compositions for parenteral use essentially without undesired histamine releasing side-effect, and wherein the content of solubilizer and other auxiliary substances is very low. This need is especially desired when developing compositions containing compounds having a very low solubility in water such as vitamin D or vitamin D analogues.

GB 899,656 discloses an aqueous vitamin D solution comprising a calcium salt and a solubilising agent. The vitamin D is in the form of a vitamin D₂ or vitamin D₃ ester of a fatty acid of not more than 8 carbon atoms. The solubilising agent may be a mono-fatty acid ester of a polyalkylene oxide, a partial ester of fatty acid and apolyhydric alcohol or an anhydride of such an alcohol etherified with polyalkylene oxide. The naturally occurring vitamin D₂ and vitamin D₃ are not particularly susceptible to degradation in an aqueous acidic environment, and while the ester group would probably be hydrolysed from the vitamin D fatty acid esters, this would not have any impact on the activity or stability of the resulting vitamin D₂ or D₃.

WO 96/36340 discloses an aqueous composition of a pH of 5.8-7.8 comprising 1α,25-dihydroxycholecalciferol (calcitriol), a solubilising agent and an antioxidant and essentially free of a buffer and a chelating agent. The solubilising agent is selected from among a wide range of substances, including dimethylacetamide, polyethylene glycol, ethanol, isopropanol, propylene glycol, dimethylsulfoxide, water, glycerin, polysorbate, polyoxyalkylene compounds, partial esters of fatty acids, partial esters of polyhydric alcohols and anhydrides of polyhydric alcohols etherified with polyalkylene oxides. While it is indicated that non-ionic solubilisers such as polyoxyalkylene compounds, partial esters of fatty acids or polyhydric alcohols, or anhydrides of polyhydric alcohols etherified with polyalkylene oxides, are preferred, and while mono-fatty acid esters of polyethylene glycol are mentioned as examples of polyoxyalkylene compounds, no specific examples of such compounds are given in WO 96/36340, and they are not indicated to be particularly preferred. It should be noted that calcitriol is not susceptible to degradation in an aqueous acidic environment and that the solubiliser has not been selected to have no negative impact on the stability of an acid labile active substance.

EP 800 825 discloses parenteral compositions comprising aqueous solubilisates of carotenoids and lipophilic vitamins in approximately equal amounts for parenteral administration to prevent or treat vitamin depletion and, with respect to carotenoids, tocopherol and ascorbic acid, as antioxidants. The object is to prevent destruction of the micellar structure and consequent precipitation of solubilised carotenoids when formulated with at least equal amounts of lipophilic vitamins. This object has been achieved by including a non-ionic emulsifier, e.g. Solutol® HS 15, in the compositions.

GB 1,123,433 discloses a non-aqueous composition comprising vitamin A and an oil and water soluble polyoxyethylene material intended as a vitamin supplement for veterinary use. The composition may additionally contain vitamin D and/or vitamin E. The only vitamin D component indicated in the description is vitamin D₂ which is not a vitamin D analogue susceptible to degradation in an aqueous acidic environment. Several types of polyoxyethylene materials are indicated in the description, including polyethylene glycol esters of fatty acids.

GB 710,817 discloses a process of producing aqueous solutions of lipophilic vitamins, in particular vitamin E and vitamin D (vitamin D₂ and D₃) by dissolving them in polyoxyethylene glycol monoricinoleate containing 30-50 oxyethylene units, and diluting this solution with water.

The present Inventors have found that a solubilized aqueous pharmaceutical composition containing seocalcitol as the drug substance can be obtained by using a relatively small amount of solubilizer and, where required or optionally, comparatively small amounts of auxiliary substances. Furthermore, such compositions are stable with respect to the chemical stability of the drug substance.

### Summary of the invention

Thus, the present invention provides an aqueous pharmaceutical composition for parenteral use, said composition comprising, as the active substance, seocalcitol [1(S),3(R)-dihydroxy-20(R)-(5'-ethyl-5'-hydroxy-hepta-1'(E),3'(E)-diene-1'-yl)-9,10-secopregna-5(Z),7(E),10(19)-triene]; a non-ionic solubilizer capable of solubilizing said active substance, the solubilizer being a low histamine-releaser, i.e. a substance which, e.g. in dogs or cats on first exposure releases at most 1/10 of histamine compared to Cremophor® EL, and containing a fatty acid ester of polyethylene glycol optionally in admixture with a polyethylene glycol and having no negative effect on the stability of the active substance in the final composition determined as described herein, and an aqueous vehicle.

The invention also relates to the use of a composition according to the present invention for the manufacture of a medicament for the prevention or treatment of cell proliferative disorders, disorders of the calcium metabolism or neoplastic diseases. Said neoplastic disease Is preferably a hepatoma, a cancer of the pancreas, mamma, colon or prostata, or a hematological neoplastic disorder.

### Detailed description of the invention

In the present context, the term "susceptible to degradation in an aqueous acidic environment" means that the degradation rate of the active substance is at least 5 times greater at an acidic pH (e.g. pH 4) than at a neutral-weakly alkaline pH (e.g. pH 8) and at a temperature of about 25°C. Said degradation has been observed with active substances such as seocalcitol showing a correlation between decreasing pH and increasing velocity constant of degradation.

Furthermore, the term "no negative effect on the stability" is intended to mean that the degradation of seocalcitol in a composition according to the invention is at the most of the same order of magnitude or less than the degradation of seocalcitol in a conventional co-solvent composition containing the same amount of seocalcitol and having the same pH as that of the composition according to the invention. A suitable co-solvent composition for use in testing any effect on the stability is the co-solvent composition described in Example 3 herein, i.e. a co-solvent composition containing ethanol-propylene glycol in a weight ratio of from about 1:5 to about 1:10.

In the present context the term "stability" is intended to mean that after 3 months storage at 40°C, a degradation of seocalcitol of at the most about 10% w/w such as at the most about 6% w/w or at the most about 3% w/w of the active substance is observed.

### Solubilizer

As mentioned above, a composition according to the invention contains a non-ionic solubilizer capable of solubilizing seocalcitol. The solubilizer contains polyoxyethylene groups and has no negative effect on the stability of seocalcitol in the final composition. The final composition is in the form of an aqueous solution. In general, a parenteral administration form in the form of a solution is very advantageous, e.g., because i) a quicker onset of the effect, ii) a better reproducibility, and iii) compared with compositions also in the the form of solutions but containing a cosolvent, less pain on the injection site after injection.

Some surfactants (i.e. substances which, at low concentrations, adsorb onto the surfaces or interfaces of a system and alter the surface or interfacial free energy and the surface or interfacial tension) also have a function as solubilizing agents.

Solubilization can be defined as the preparation of a thermodynamically stable isotropic solution of a substance normally insoluble or very slightly soluble in a given solvent by the introduction of an additional amphiphilic component or components. Solubilization normally takes place by formation of micelles (i.e. the solubilizer must be present in a concentration above their critical micelle concentration) and the location of the solubilized molecule in a micelle may be *inter alia* i) on the surface at the micelle-solvent interface, ii) between the hydrophilic groups, or iii) in the micelle inner core.

A non-ionic solubilizer is a solubilizer where the hydrophile part of the solubilizer carries no charge but derives its water solubility from highly polar groups such as hydroxyl or polyoxyethylene groups.

A non-ionic solubilizer for use in pharmaceutical compositions according to the Invention is typically a low histamine-releaser, I.e. a substance which e.g. in dogs and cats on first exposure releases at the most 1/10 of histamine compared to Cremophor ® EL. Methods for use in determining the histamine release are found e.g. in Lorenz, W. et al. in Agents and Actions, 1982, **12 1/2,** 64-80 and in Ennis, M. et al. in Agents and Actions, 1985, **16 3/4,** 265-268.

A non-ionic solubilizer for use in pharmaceutical compositions according to the invention contains typically a hydrophobic part and a hydrophilic part, such as about 70% w/w of a hydrophobic part and about 30% w/w of a hydrophilic part.

A non-ionic solubilizer suitable for use in a pharmaceutical composition according to the invention is a polyethylene glycol ester optionally in admixture with a polyethylene glycol. The polyethylene glycol and/or the polyethylene glycol part of the polyethylene glycol ester generally have a molecular weight of from about 200 to about 4000.

More specifically, the polyethylene glycol ester is a polyethylene glycol hydroxystearate such as a polyethylene glycol 12-hydroxystearate and the polyethylene glycol hydroxystearate may be or is a monoester, a diester or a mixture thereof.

As mentioned above, a pharmaceutical composition according to the invention may further comprise polyethylene glycol. In preferred embodiments of the invention the polyethylene glycol and/or the polyethylene glycol part of the polyethylene glycol ester suitable for use in compositions according to the invention have a molecular weight of from about 200 to about 800.

An especially suitable solubilizer for use in a pharmaceutical composition according to the invention is a solubilizer comprising polyethylene glycol 660 hydroxystearate such as, e.g. Solutol® HS15. Solutol® HS15 is manufactured by BASF, cf. Technical Information ME 151 e (977) July 1998 (MPM) from BASF Fine Chemicals, and contains about 65-70% of polyglycolester of 12-hydroxystearic acid as a hydrophobic part and about 25-30% of polyethylene glycol as a hydrophilic part. The main fatty acid component of Solutol® HS15 is 12-hydroxystearic acid, where also stearic and palmitic acid are present in detectable amounts. Free polyethylene glycol is present as well as its mono- and di-esters, and a further 2-3% of the 12-hydroxy group has been etherified with ethylene oxide. Solutol® HS15 has been proven to be especially suitable for use as a solubilizer in compositions according to the invention. However, other combinations of polyethylene glycolesters of 12-hydroxystearic acid, stearic acid, palmitic add and/or polyethylene glycol as well as use of polyethylene glycolesters and/or polyethylene glycols having other molecular weights than those relevant for Solutol® HS15, are of course within the scope of the present invention.

In general, a non-ionic solubilizer for use in compositions according to the invention has a HLB value of at least about 12.

The concentration of the non-ionic solubilizer in a composition according to the invention is intended to be as low as possible (so as to avoid any significant negative influence after administration), normally within a range of from 0.001% to 30% w/v such as, e.g. from 0.005% to 25% w/v, from 0.01 to 20% w/v, from 0.01 to 15%, from 0.01% to 10% w/v, from 0.01% to 8% w/v, from 0.05% to 6% w/v, from 0.05% to 5% w/v, from 0.1% to 3% w/v, from 0.2% to 2% w/v. from 0.3% to 1.5% w/v or from 0.4% to 1% w/v such as, e.g. about 0.5% w/v.

### Aqueous vehicle

A composition according to the invention comprises an aqueous vehicle. The aqueous vehicle contains of course water, but it may furthermore also contain pH adjusting agents, stabilizing agents, solubilizing agent (cf. above), isotonic adjusting agents, solvents (e.g. organic solvents; as discussed below, a composition according to the invention is most suitably formulated without any content of organic solvent, but in some cases it may be appropriate to include an organic solvent as well), etc. The concentration of water in a composition according to the invention is normally at least about from 65% to 99% w/w.

In contrast to prior art compositions, it is not mandatory that the aqueous vehicle of a composition according to the invention contains any chelating agent, ethanol and/or another organic solvent.

The pH in a composition according to the present invention is normally within the physiological range, i.e. it is in a range of from 7.0 to 9.0 such as, e.g. from 7.0 to 8.5, from 7.0 to 8.0, from 7.0 to 7.8, from 7.4 to 7.6 such as, e.g., 7.4 or 7.5.

In interesting embodiments of the invention, a composition comprises from 0.0001% to 0.01% w/v of Seocalcitol and from 0.1% to 1.5% w/v of Solutol® HS 15 or from 0.0005% to 0.008% w/v of Seocalcitol and from 0.2% to 1.0% w/v of Solutol® HS 15.

A pharmaceutical composition according to the invention may further comprise an antioxidant. Especially suitable are antioxidants which have a solubility in water which is at least 1.5 times greater than their solubility in the non-ionic solubilizer. Relevant examples are sodium ascorbate, sodium bisulfite, sodium sulfite and mixtures thereof.

In the present context it is important that the antioxidant is soluble in water. In a composition according to the invention, a part of the seocalcitol is present in the aqueous medium in soluble form and any oxidation takes place in this medium. On the contrary, if the antioxidant is fat-soluble, it is contemplated that the antioxidant and seocalcitol would compete for the space within the micelles resulting in a decrease in the solubility of seocalcitol.

The concentration of an antioxidant - such as, e.g., sodium ascorbate - when present in a composition according to the invention, is from 0.2% to 3% w/v such as, e.g., from 0.3% to 2.5% w/v or from 0.4 to 2.0% w/v such as, e.g., about 0.5% w/v in the final composition.

As appears from the Examples herein, a specific embodiment of the invention is a pharmaceutical composition containing

| | |
|---|---|
| 10 µg | seocalcitol |
| 15.4 mg | disodium phosphate dihydrate |
| 2 mg | sodium dihydrogen phosphate dihydrate |
| 0.8 mg | sodium chloride |
| 5 mg | sodium ascorbate |
| 5 mg | Solutol® HS 15 |
| ad 1 ml water for injection. | |

Use of the compositions according to the invention

A composition according to the present invention containing seocalcitol, is suitable for use in the treatment and/or prophylaxis of:
i) diseases or conditions characterized by abnormal cell differentiation and/or cell proliferation such as, e.g., psoriasis and other disturbances of keratinisation, HIV-associated dermatoses, wound healing, neoplastic diseases and cancer. Specific examples of neoplastic diseases are hepatomas, pancreas, mamma, colon and prostata cancer as well as hematological neoplastic disorders and skin cancer, and
ii) diseases of, or imbalance in, the immune system such as host-versus-graft and graft-versus-host reaction and transplant rejection, and auto-immune diseases such as discoid and systemic lupus erythematosus, diabetes mellitus and chronic dermatoses of auto-immune type e.g. scteroderma and pemphigus vulgaris, and
iii) inflammatory diseases such as rheumatoid arthritis
as well as in the treatment and/or prophylaxis of a number of other diseases or disease states, including hyperparathyroidism, particularly secondary hyperparathyroidism associated with renal failure, cognitive impairment or senile dementia (Alzheimer's disease) and other neurodegenerative diseases, hypertension, acne, alopecia, skin atrophy e.g. steroid induced skin atrophy, skin ageing, including photo-ageing, and in promoting osteogenesis and treating/preventing osteoporosis and osteosmalacia.

The composition according to the invention is especially suited for treatment of cell proliferative disorders; disorders of the calcium metabolism, such as renal osteodystrophy, hypoparathyreoidism, vitamin D resistant hypophosphataemic rachitis, or osteomalacia; or neoplastic diseases, such as hepatomas, cancer of the pancreas, mamma, colon or prostata, or a hematological neoplastic disorder; where the method of treatment comprises administering to a patient in need thereof a composition according to the invention.

### Administration routes for the compositions according to the invention

As mentioned in the introductory part, a composition according to the invention is intended for parenteral use, i.e. for use by injection into e.g. an animal or human body. The application may be by the intraveneous, intramuscular and subcutaneous route, the intraveneous route being preferred for compositions suitable for use in connection with neoplastic disorders.

However, whenever relevant, compositions according to the invention may also be suitable for use by other administration routes such as, e.g., the oral route, the topical route and the nasal route. In such cases, a person skilled in the art can make any necessary adjustments with respect to the concentration of seocalcitol and with respect to the other ingredients included in the composition.

Apart from seocalcitol and an aqueous vehicle, a composition according to the invention may contain pharmaceutically or cosmetically acceptable excipients such as, e.g., preservatives like e.g. parabens, such as methyl, ethyl or propyl p-hydroxybenzoate, benzalkonium chloride and benzylalcohol, antioxidants as discussed above, chelating agents, e.g. EDTA, citric acid and phosphoric acid, flavouring agents, gelling agents such as, e.g., water-soluble cellulose derivatives and carbomers.

A composition according to the invention may be presented in the form of a unit dose (e.g. in ampoules) or a multiple-unit dose composition (i.e. in vials containing two or more doses).

### Dosage

In general the concentration of seocalcitol in the composition and the dosis will depend on the condition to be treated or prevented and the desired or necessary administration frequency. The concentration of seocalcitol in a pharmaceutical composition depends on the nature of the second compound in question, its potency, the severity of the disease to be prevented or treated, and the age and condition of the patient. Methods applicable to selecting relevant concentrations of the active substance in the pharmaceutical compositions are well known to a person skilled in the art and may be performed according to established guidelines for good clinical practice (GCP) or Investigational New Drug Exemption ("IND") regulations as described in e.g. CPMC/E.U. Guidelines for Good Clinical Practice 95/135. A person skilled in the art would, by use of the methods described in standard textbooks, guidelines and regulations as described above as well as common general knowledge within the field, be able to select the exact dosage regimen to be implemented for any active substance and dosage form using merely routine experimentation procedures.

In embodiments containing Seocalcitol as the active substance, a dosage of about 1-100 µg is suitable.

As will be understood, details and particulars concerning the composition aspects of the Invention will be the same as or analogous to the details and particulars concerning the other aspects of the invention, and this means that wherever appropriate, the statements above concerning a pharmaceutical composition apply mutatis mutandis to all aspects of the invention.

The invention is illustrated further in the following, non-limiting examples.

### EXAMPLES

### Example 1

### Preparation of an aqueous pharmaceutical composition according to the invention

An aqueous solution for parenteral administration is prepared as follows

| Ingredients (all pharmacopoeial grade) | per ml |
|---|---|
| Seocalcitol (active substance) | 10 µg |
| Disodium phosphate dihydrate (buffer) | 15.4 mg |
| Sodium dihydrogen phosphate dihydrate (buffer) | 2 mg |
| Sodium chloride | 0.8 mg |
| Sodium ascorbate (antioxidant) | 5 mg |
| Solutol® HS 15 from BASF (solubilizer) | 5 mg |
| Water for injection | ad 1 ml |

Solutol® HS 15 is dissolved in the water for injection by heating it to a temperature of at the most 80°C. A cover of nitrogen is applied. The buffer substances and the sodium chloride are added and then the solution is cooled to at the most 30°C. Then sodium ascorbate is added and, finally, seocalcitol is dissolved in the solution obtained.

The solution is subjected to sterile filtration and is autoclaved at an appropriate time-temperature condition.

### Example 2

### Chemical stability of seocalcitol In aqueous pharmaceutical compositions

The chemical stability of seocalcitol in aqueous solutions was investigated in order to determine whether pharmaceutically acceptable excipients generally used have any influence on the stability. pH in the present aqueous solutions was adjusted to pH=7.5. In the presence of ascorbate but without N₂ cover the pH value decreased to 7.1 - 7.2 within a short period of time.

Seocalcitol is a vitamin D analogue which is susceptible to degradation in an aqueous acidic environment. Thus, the degradation rate is lowered by a factor of about 25 when pH is shifted from acidic pH to neutral-weakly alkaline pH. When acid is present, seocalcitol may be degraded by an allyl inversion, or seocalcitol may be decomposed.

The decay of seocalcitol was monitored by use of a HPLC method employing a 125 mm x 4 mm column packed with LiChrospher RP-18, 5 µm and a mobile phase of acetonitril : 0.01 M phosphate buffer pH 6.0 (60:40). The flow rate was 2 ml/min, 500 µl of a sample was injected and the effluent was monitored at UV-264 nm.

The influence on the degradation of seocalcitol was determined for the following excipients:

| Solubilizer: | Tween 20 and Solutol® HS 15 |
|---|---|
| Concentration of solubilizer: | 5 mg/ml and 10 mg/ml |
| Sodium ascorbate (antioxidant) | 0 mg/ml and 10 mg/ml |
| EDTA (chelating agent) | 0 mg/ml and 1 mg/ml |
| Nitrogen cover | - (0) and + (1) |

The following experiments were performed:

**Table 1**

| Degradation in % after storage for 3 months at 25°C | | | | | | |
|---|---|---|---|---|---|---|
| Batch No. | solubilizer | solubilizer conc. mg/ml | Ascorbate mg/ml | EDTA mg/ml | Nitrogen | Degr. (%) at 25°C |
| 9625813 | Tween 20 | 10 | 10 | 0 | 0 | 0 |
| 9625814 | Tween 20 | 5 | 0 | 0 | 0 | 13.57 |
| 9625815 | Tween 20 | 10 | 10 | 1 | 1 | 0.68 |
| 9625816 | Tween 20 | 5 | 10 | 0 | 1 | 0 |
| 9625817 | Tween 20 | 5 | 10 | 1 | 0 | 0.3 |
| 9625818 | Tween 20 | 5 | 0 | 1 | 1 | 17.4 |
| 9625819 | Tween 20 | 10 | 0 | 1 | 0 | 27.05 |
| 9625820 | Tween 20 | 10 | 0 | 0 | 1 | 8.73 |
| 9617911 | Solutol HS15 | 10 | 10 | 0 | 0 | 0 |
| 9617912 | Solutol HS15 | 5 | 0 | 0 | 0 | 5.79 |
| 9617913 | Solutol HS15 | 10 | 10 | 1 | 1 | 0.35 |
| 9617914 | Solutol HS15 | 5 | 10 | 0 | 1 | 0 |
| 9617915 | Solutol HS15 | 5 | 10 | 1 | 0 | 1.27 |
| 9617916 | Solutol HS15 | 5 | 0 | 1 | 1 | 5.42 |
| 9617917 | Solutol HS15 | 10 | 0 | 1 | 0 | 8.62 |
| 9617918 | Solutol HS15 | 10 | 0 | 0 | 1 | 3.17 |

**Table 2**

| Degradation in % after 3 months at 25°C in formulations without ascorbate | | | | | | |
|---|---|---|---|---|---|---|
| Batch No. | solubilizer | Solubilizer conc. mg/ml | Ascorbate mg/ml | EDTA mg/ml | Nitrogen | Degr. (%) at 25°C |
| 9625814 | Tween 20 | 5 | 0 | 0 | 0 | 13.57 |
| 9625818 | Tween 20 | 5 | 0 | 1 | 1 | 17.4 |
| 9625819 | Tween 20 | 10 | 0 | 1 | 0 | 27.05 |
| 9625820 | Tween 20 | 10 | 0 | 0 | 1 | 8.73 |
| 9617912 | Solutol HS15 | 5 | 0 | 0 | 0 | 5.79 |
| 9617916 | Solutol HS15 | 5 | 0 | 1 | 1 | 5.42 |
| 9617917 | Solutol HS15 | 10 | 0 | 1 | 0 | 8.62 |
| 9617918 | Solutol HS15 | 10 | 0 | 0 | 1 | 3.17 |

**Table 3**

| Degradation in % after 3 months at 25°C in formulations with ascorbate | | | | | | |
|---|---|---|---|---|---|---|
| Batch No. | Solubilizer | Solubilizer conc. mg/ml | Ascorbate mg/ml | EDTA mg/ml | Nitrogen | Degr. (%) at 25°C |
| 9625813 | Tween 20 | 10 | 10 | 0 | 0 | 0 |
| 9625815 | Tween 20 | 10 | 10 | 1 | 1 | 0.66 |
| 9625816 | Tween 20 | 5 | 10 | 0 | 1 | 0 |
| 9625817 | Tween 20 | 5 | 10 | 1 | 0 | 0.3 |
| 9617911 | Solutol HS15 | 10 | 10 | 0 | 0 | 0 |
| 9617913 | Solutol HS15 | 10 | 10 | 1 | 1 | 0.35 |
| 9617914 | Solutol HS15 | 5 | 10 | 0 | 1 | 0 |
| 9617915 | Solutol HS15 | 5 | 10 | 1 | 0 | 1.27 |

### Degradation after 3 months at 25°C (Table 1-3):

The results indicate that solutions containing Solutol® HS 15 are more stable than solutions containing Tween 20 as solubilizer. Thus, e.g. the decay of the seocalcitol in solutions without any content of ascorbate, EDTA and without any nitrogen cover but containing 5 mg/ml of Tween 20 or Solutol® HS 15, respectively, was 13.57% and 5.79%, respectively.

Furthermore, the results indicate that EDTA has a negative effect on the stability of seocalcitol in solutions containing Solutol® HS 15 as solubilizer and ascorbate (see e.g. Table 3 and compare the results from batch No. 9615817 with the results from batch No. 9617915). Normally, EDTA is used in solutions containing substances which are susceptible to oxidative degradation in order to enable a complexation of any metal ions which may accelerate the oxidative degradation. However, the results obtained indicate that compositions containing Solutol® HS 15 may be sufficiently stabilized against oxidative degradation by using ascorbate and, thus, avoiding any further stabilizing agent present in the solution. In other words, the results indicate that it is possible to formulate a composition in soluble form with a content of very few pharmaceutically acceptable excipients. Such compositions are of course especially needed in those cases where the compositions are intended for injection directly into the circulatory system (any side effect of an excipient becomes of course more pronounced when a composition containing the excipient is injected directly into the circulatory system).

In short, the results show that solutions containing sodium ascorbate lead to significantly less degradation of seocalcitol. The same applies to solutions containing Solutol® HS 15, whereas solutions containing EDTA have no positive effect on the stability of Seocalcitol.

### Overall conclusion

To ensure an appropriate stability of seocalcitol, the presence of sodium ascorbate Is necessary. At 25°C, Solutol® HS 15 has a much better effect on the stability than Tween 20, especially for solutions without sodium ascorbate.

### Example 3

Improvement of the chemical stability of seocalcitol - comparison of the stability of two different compositions of Seocalcitol

The stability of the compositions from Example 2 containing seocalcitol solubilized in an aqueous vehicle without any content of organic solvent and containing Solutol® HS 15 as a solubilizer (Table 4) was compared with the stability of compositions of seocalcitol containing a co-solvent of ethanol-propylene glycol (Table 5) and having a content of seocalcitol of 10 or 30 µg. The compositions containing co-solvent were adjusted to a specific pH value (in a range of 6.8 - 9.2) in order to investigate the influence of pH on the stability of the compositions. An example of the constitution of a co-solvent composition having a pH of about 9 is given in the following:

| | |
|---|---|
| Seocalcitol | 10 or 30 µg |
| Disodium phosphate dihydrate | 1.8 mg |
| Sodium chloride | 3.88 mg |
| Benzyl alcohol | 15.675 mg |
| Ethanol 99.9% | 79.2 mg |
| Propylene glycol | 414 mg |
| Water for injection to make 1 ml | |

The stability of the various compositions was determined after 3 months of storage at a temperature of 40°C and the results are given in the following Tables 4-5.

### Seocalcitol injection

### Stability data for solubilized and cosolvent formulations

**Table 4**

| Solutol® HS15 | | | | |
|---|---|---|---|---|
| Batch No. | pH | Ascorbate added mg/ml | Degradation (%) after 3 months at 40°C | Comments |
| 9617911 | 7.2 | 10 | 1.09 | 30mcg active/ml; without Nitrogen |
| 9617913 | 7.4 | 10 | 1.5 | 30mcg active/ml; with Nitrogen; + EDTA |
| 9617914 | 7.4 | 10 | 0 | 30mcg active/ml; with Nitrogen |
| 9617915 | 7.2 | 10 | 3.5 | 30mcg active/ml; without Nitrogen + EDTA |
| 9634916 | 7.5 | 5 | 0 | 10mcg active/ml; without Nitrogen |
| 9634917 | 7.2 | 5 | 0.14 | 30mcg active/ml; without Nitrogen |
| 9822012 | 7.5 | 5 | 2.2 | 10mcg active/ml; with Nitrogen; autoclaved |
| 9634915 | 7.3 | 1 | 0 | 10mcg active/ml; without Nitrogen |
| 9634918 | 7.3 | 1 | 2.2 | 30mcg active/ml; without Nitrogen |

**Table 5**

| Cosolvent | | | |
|---|---|---|---|
| Batch No. | pH of the water part | Degr. (%) after 3 months at 40°C | Comments |
| 9524911 | 6.83 | 16 | 10mcg active/ml |
| 9524912 | 7.55 | 6.23 | 10mcg active/ml |
| 9524913 | 9.16 | 0.39 | 10mcg active/ml |
| 9524214 | 6.78 | 6.18 | 10mcg active/ml |
| 9608211 | 9.04 | 1.59 | 10mcg active/ml |
| 9608711 | 8.97 | 1.23 | 10mcg active/ml |
| 9626611A | 8.99 | 2.66 | 10mcg active/ml; autoclaved |
| 9626611B | 8.99 | 2.07 | 10mcg active/ml; autoclaved |
| 9530211 | 7.5 | 4.53 | 30mcg active/ml |
| 9530212 | 7.58 | 3.73 | 30mcg active/ml |
| 9605911 | 9.01 | 2.2 | 30mcg active/ml |
| 9606511 | 9.17 | 0.78 | 30mcg active/ml |

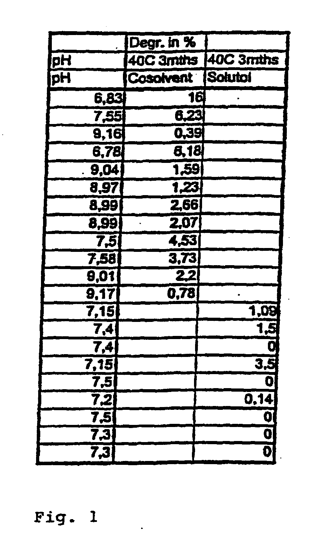

The results show that compositions containing co-solvent are more susceptible to degradation than compositions without co-solvent (i.e. compositions containing a solubilizer, especially compositions containing Solutol® HS 15). Thus, in order to obtain a relatively low degree of degradation of seocalcitol, the pH of the compositions containing co-solvent should be about 9. In contrast thereto, a pH of about 7-7.5 of compositions containing Solutol® HS 15 is acceptable in order to obtain a low degree of degradation of seocalcitol (see Fig. 1).

In other words, by employment of a solubilizer like Solutol® HS 15, it is possible to obtain aqueous solutions of active substances having a relatively low solubility in water, and the compositions may be without any content of organic solvent (i.e. it is possible to obtain compositions without any content of organic solvent(s) or it is possible to significantly reduce the high content of organic solvent(s), which are present in a traditional co-solvent composition). Compositions according to the invention, i.e. compositions containing a solubilizer, may very advantageously be employed for parenteral use compared with compositions containing an organic solvent (e.g. in a relatively high amount) because an organic solvent in compositions intended for parenteral administration normally leads to side effects like pain (in connection with the administration) and to necrosis of tissue.

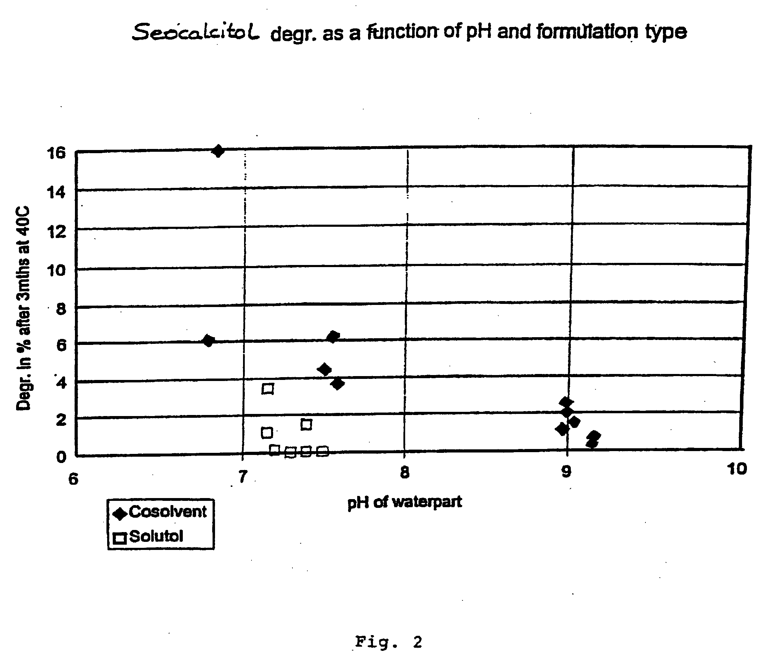

Furthermore, the results indicate that Solutol® HS 15 has a stabilizing effect on seocalcitol. At pH 7.5, the degradation of seocalcitol after 3 months storage at 40°C is 2.2% for a composition containing Solutol® HS 15 whereas it is 4.53% for a composition containing a co-solvent (i.e. without any solubilizer). With respect to compositions containing a co-solvent, an increase of pH to above about 9 seems to be necessary in order to obtain a degradation of seocalcitol below 1% after 3 months storage at 40°C (see Fig. 2).

### Conclusion

In other words, at the pH employed (pH 7.5) Solutol® HS 15 not only functions as a solubilizer but it also appears to function as a stabilizing agent and improves the chemical stability of seocalcitol.

## Claims

1. An aqueous pharmaceutical composition for parenteral use, said composition comprising
i) seocalcitol [1(S),3(R)-dihydroxy-20(R)-(5'-ethyl-5'-hydroxy-hepta-1'(E),3'(E)-diene-1'-yl)-9,10-secopregna-5(Z),7(E),10(19)-triene],
ii) a non-ionic solubilizer capable of solubilizing said active substance, the solubilizer being a low histamine-releaser, i.e. a substance which e.g. in dogs and cats on first exposure releases at the most 1/10 of histamine compared to Cremophor ® EL, and containing a fatty acid ester of polyethylene glycol optionally in admixture with a polyethylene glycol and having no negative effect on the stability of the active substance in the final composition determined as described herein, and
iii) an aqueous vehicle.

2. A pharmaceutical composition according to claim 1, wherein the non-ionic solubilizer is a fatty acid ester of polyethylene glycol in admixture with a polyethylene glycol and wherein the polyethylene glycol and/or the polyethylene glycol part of the fatty acid polyethylene glycol ester has a molecular weight of from 200 to 4000.

3. A pharmaceutical composition according to claim 2, wherein the fatty acid polyethylene glycol ester is a polyethylene glycol hydroxystearate such a polyethylene glycol 12-hydroxystearate.

4. A pharmaceutical composition according to claim 3, wherein the polyethylene glycol hydroxystearate is a monoester, a diester or a mixture thereof.

5. A pharmaceutical composition according to any one of claims 2-4, wherein the polyethylene glycol and/or the polyethylene glycol part of the polyethylene glycol ester have a molecular weight of from 200 to 800.

6. A pharmaceutical composition according to any one of claims 1-5, wherein said non-ionic solubilizer comprises polyethylene glycol 660 hydroxystearate.

7. A pharmaceutical composition according to claim 6, wherein the non-ionic solubilizer is Solutol® HS 15 as described in Technical Information ME 151 e (977) July 1998 (MPM) from BASF Fine Chemicals.

8. A pharmaceutical composition according to claim 1, wherein the non-ionic solubilizer has an HLB value ≥ 12.

9. A pharmaceutical composition according to claim 1, wherein the non-ionic solubilizer is Solutol® HS 15 or polyethylene glycol 660 hydroxystearate.

10. A pharmaceutical composition according to any one of claims 1-9, wherein the concentration of the non-ionic solubilizer is in a range of from 0.001% w/v to 30% w/v; preferably from 0.005% w/v to 25% w/v; or preferably from 0.01% w/v to 20% w/v, preferably to 15%, more preferably to 10% w/v, most preferably to 8% w/v; more preferably from 0.05% w/v to 6% w/v, preferably to 5% w/v; or more preferably from 0.1% w/v to 3% w/v, or more preferably from 0.2% w/v to 2% w/v; and most preferably from 0.3% w/v to 1.5% w/v; or most preferably from 0.4% w/v to 1% w/v; or the concentration of said non-ionic solubilizer is preferably about 0.5% w/v,

11. A pharmaceutical composition according to claim 10, the composition comprising from 0.0001% w/v to 0.01% w/v of seocalcitol and from 0.1% w/v to 1.5% w/v of Solutol® HS 15, preferably from 0.0005% w/v to 0.008% w/v of seocalcitol and from 0.2% to 1.0% w/v of Solutol® HS 15.

12. A pharmaceutical composition according to any one of claims 1-11 further comprising less than 1 % w/w, preferably less than 0.05% w/w, more preferably about 0.025% w/w, most preferably 0.01% w/w of a chelating agent.

13. A pharmaceutical composition according to any one of claims 1-12, wherein the aqueous vehicle contain less than 5% w/w , preferably less than 4% w/w, preferably less than 3% w/w, more preferably less than 2% w/w, or most preferably less than 1% w/w of an organic solvent.

14. A pharmaceutical composition according to claim 13, wherein the organic solvent is polyethylene glycol or polypropylene glycol.

15. A pharmaceutical composition according to any one of claims 1-14, wherein the pH of the composition is in the range of from 7.0 to 9.0, preferably from 7.0 to 8.5, more preferably from 7.0 to 8.0, most preferably from 7.0 to 7.8.

16. A pharmaceutical composition according to any one of claims 1-15 further comprising an antioxidant which has a solubility in water which is at least 1.5 times greater than its solubility in the non-ionic solubilizer.

17. A pharmaceutical composition according to claim 16, wherein the antioxidant is selected from the group consisting of sodium ascorbate, sodium bisulfite, and sodium sulfite.

18. A pharmaceutical composition according to claim 17, wherein the concentration of sodium ascorbate in the final composition is from 0.2% w/v to 3% w/v, preferably from 0.3% w/v to 2.5% w/v, more preferably from 0.4 % w/v to 2.0% w/v, most preferably about 0.5% w/v.

19. Use of a pharmaceutical composition according to any one of claims 1-18 for the manufacture of a medicament for the prevention or treatment of cell proliferative disorders, disorders of calcium metabolism or neoplastic diseases.

20. The use according to claim 19, wherein said neoplastic diseases are hepatomas; cancer of the pancreas, mamma, colon or prostate; or a hematological neoplastic disorder.

21. The use according to claim 19, wherein said disorders of the calcium metabolism are renal osteodystrophy, hypoparathyreoidism, vitamin D resistant hypophosphataemic rachitis, or osteomalacia.

## Patentansprüche

1. , Wässrige pharmazeutische Zusammensetzung zur parenteralen Verwendung, wobei die Zusammensetzung umfasst:
i) Seocalcitol [1 (S),3(R)-Dihydroxy-20(R)-(5'-ethyl-5'-hydroxy-hepta-1'(E),3'(E)-dien-1-yl)-9,10-secopregna-5(Z),7(E),10(19)-trien],
ii) einen nichtionischen Lösungsvermittler, der die aktive Substanz zu solubilisieren vermag, wobei der Lösungsvermittler eine Substanz mit geringer Histaminfreisetzung ist, d.h. eine Substanz, die beispielsweise in Hunden und Katzen höchstens 1/10 des Histamins im Vergleich zu Cremophor® EL freisetzt, der Lösungsvermittler einen Polyethylenglykol-Fettsäureester gegebenenfalls im Gemisch mit einem Polyethylenglykol enthält und der Lösungsvermittler keine negative Wirkung auf die Stabilität der aktiven Substanz in der fertigen Zusammensetzung - wie hier bestimmt - hat, und
iii) ein wässriges Vehikel.

2. Pharmazeutische Zusammensetzung nach Anspruch 1, worin der nichtionische Lösungsvermittler ein Polyethylenglykol-Fettsäureester im Gemisch mit einem Polyethylenglykol ist und worin das Polyethylenglykol und/oder der Polyethylenglykolteil des Fettsäurepolyethylenglykolesters ein Molekulargewicht von 200 bis 4000 besitzt.

3. Pharmazeutische Zusammensetzung nach Anspruch 2, worin der Polyethylenglykol-Fettsäureester ein Polyethylenglykolhydroxystearat, z.B. ein Polyethylenglykol-12-hydroxystearat, ist.

4. Pharmazeutische Zusammensetzung nach Anspruch 3, worin das Polyethylenglykolhydroxystearat ein Monoester, ein Diester oder ein Gemisch davon ist.

5. Pharmazeutische Zusammensetzung nach einem der Ansprüche 2 bis 4, worin das Polyethylenglykol und/oder der Polyethylenglykolteil des Polyethylenglykolesters ein Molekulargewicht von 200 bis 800 besitzt.

6. Pharmazeutische Zusammensetzung nach einem der Ansprüche 1 bis 5, worin der nichtionische Lösungsvermittler ein Polyethylenglykol-660-hydroxystearat umfasst.

7. Pharmazeutische Zusammensetzung nach Anspruch 6, worin der nichtionische Lösungsvermittler Solutol® HS 15 ist, das in der technischen Information ME 151 e (977) Juli 1998 (MPM) der BASF Fine Chemicals beschrieben ist.

8. Pharmazeutische Zusammensetzung nach Anspruch 1, worin der nichtionische Lösungsvermittler einen HLB-Wert ≥ 12 besitzt.

9. Pharmazeutische Zusammensetzung nach Anspruch 1, worin der nichtionische Lösungsvermittler Solutol® HS 15 oder Polyethylenglykol-660-hydroxystearat ist.

10. Pharmazeutische Zusammensetzung nach einem der Ansprüche 1 bis 9, worin die Konzentration des nichtionischen Lösungsvermittlers im Bereich von 0,001 % G/V bis 30 % G/V; vorzugsweise im Bereich von 0,005 G/V bis 25 % G/V; oder vorzugsweise im Bereich von 0,01 % GN bis 20 % G/V, bevorzugt bis 15 %, bevorzugter bis 10 % G/V, am bevorzugtesten bis 8 % G/V; bevorzugter im Bereich von 0,05 % G/V bis 6 % GN, bevorzugt bis 5 % G/V; oder bevorzugter im Bereich von 0,1 % G/V bis 3 % G/V, oder bevorzugter im Bereich von 0,2 % G/V bis 2 % G/V; und am bevorzugtesten im Bereich von 0,3 % G/V bis 1,5 % G/V; oder am bevorzugtesten im Bereich von 0,4 % G/V bis 1 % G/V liegt; oder die Konzentration des nichtionischen Lösungsvermittlers vorzugsweise etwa 0,5 % G/V beträgt.

11. Pharmazeutische Zusammensetzung nach Anspruch 10, wobei die Zusammensetzung 0,0001 %G/V bis 0,01 % G/V Seocalcitol und 0,1 % G/V bis 1,5 % GN Solutol® HS 15, vorzugsweise 0,0005 % G/V bis 0,008 % G/V Seocalcitol und 0,2 % bis 1,0 % GN Solutol® HS 15 umfasst.

12. Pharmazeutische Zusammensetzung nach einem der Ansprüche 1 bis 11, die weiterhin weniger als 1 % G/G, vorzugsweise weniger als 0,05 % G/G, bevorzugter 0,025 % G/G, am bevorzugtesten 0,01 % G/G eines chelatisierenden Agens umfasst.

13. Pharmazeutische Zusammensetzung nach einem der Ansprüche 1 bis 12, worin das wässrige Vehikel weniger als 5 % G/G, vorzugsweise weniger als 4 % G/G, vorzugsweise weniger als 3 % G/G, bevorzugter weniger als 2 % G/G, oder am bevorzugtesten weniger als 1 % G/G eines organischen Lösungsmittels umfasst.

14. Pharmazeutische Zusammensetzung nach Anspruch 13, worin das organische Lösungsmittel Polyethylenglykol oder Polypropylenglykol ist.

15. Pharmazeutische Zusammensetzung nach einem der Ansprüche 1 bis 14, worin der pH der Zusammensetzung im Bereich von 7,0 bis 9,0, vorzugsweise im Bereich von 7,0 bis 8,5, bevorzugter im Bereich von 7,0 bis 8,0, am bevorzugtesten im Bereich von 7,0 bis 7,8 liegt.

16. Pharmazeutische Zusammensetzung nach einem der Ansprüche 1 bis 15, die weiterhin ein Antioxidans umfasst, das eine Löslichkeit in Wasser aufweist, die wenigstens 1,5-mal größer ist als die Löslichkeit des nichtionischen Lösungsvermittlers.

17. Pharmazeutische Zusammensetzung nach Anspruch 16, worin das Antioxidans ausgewählt ist unter Natriumascorbat, Natriumbisulfit und Natriumsulfit.

18. Pharmazeutische Zusammensetzung nach Anspruch 17, worin die Konzentration des Natriumascorbats in der fertigen Zusammensetzung 0,2 % GN bis 3 % G/V, vorzugsweise 0,3 % G/V bis 2,5 % G/V, bevorzugter 0,4 % G/V bis 2,0 % G/V, am bevorzugtesten etwa 0,5 % GN beträgt.

19. Verwendung einer pharmazeutischen Zusammensetzung nach einem der Ansprüche 1 bis 18 bei der Herstellung eines Medikaments zur Prävention oder Behandlung zellproliferativer Erkrankungen, Erkrankungen des Calcium-Metabolismus oder neoplastischer Erkrankungen.

20. Verwendung nach Anspruch 19, wobei die neoplastische Erkrankung ein Hepatom; Pankreas-, Brust-, Colon- oder Prostatakrebs; oder eine hämatologische neoplastische Erkrankung ist.

21. Verwendung nach Anspruch 19, wobei die Erkrankung des Calcium-Metabolismus renale Osteodystrophie, Hyperparathyreodismus, Vitamin-Dresistente hypophosphatämische Rachitis oder Osteomalazie ist.

## Revendications

1. Composition pharmaceutique aqueuse à usage parentéral, ladite composition comprenant
i) du séocalcitol [1(S),3(R)-dihydroxy-20(R)-(5'-éthyl-5'-hydroxy-hepta-1'(E),3'(E)-dién-1'-yl)-9,10-secopregna-5(Z),7(E),10(19)-triène].
ii) un solubilisant non ionique capable de solubiliser ladite substance active, le solubilisant étant un agent libérant peu d'histamine, c'est-à-dire une substance qui, par exemple chez les chiens et les chats, à la première exposition, libère au plus 1/10 d'histamine par rapport à Cremophor® EL et contenant un ester d'acide gras et de polyéthylèneglycol éventuellement en mélange avec un polyéthylèneglycol et n'ayant pas d'effet négatif sur la stabilité de la substance active dans la composition finale déterminée comme décrit ici, et
iii) un véhicule aqueux.

2. Composition pharmaceutique selon la revendication 1, où le solubilisant non ionique est un ester d'acide gras et de polyéthylèneglycol en mélange avec un polyéthylèneglycol et où le polyéthylèneglycol et/ou la partie polyéthylèneglycol de l'ester d'acide gras et de polyéthylèneglycol ont une masse moléculaire de 200 à 4 000.

3. Composition pharmaceutique selon la revendication 2, où l'ester d'acide gras et de polyéthylèenglycol est un hydroxystéarate de polyéthylèneglycol comme l'hydroxystéarate de polyéthylèneglycol 12.

4. Composition pharmaceutique selon la revendication 3, où l'hydroxystéarate de polyéthylèneglycol est un monoester, un diester ou un mélange de ceux-ci.

5. Composition pharmaceutique selon l'une quelconque des revendications 2-4 où le polyéthylèneglycol et/ou la partie polyéthylèneglycol de l'ester de polyéthylèneglycol ont une masse moléculaire de 200 à 800.

6. Composition pharmaceutique selon l'une quelconque des revendications 1-5 où ledit solubilisant non ionique comprend de l'hydroxystéarate de polyéthylèneglycol 660.

7. Composition pharmaceutique selon la revendication 6 où le solubilisant non ionique est Solutol® HS 15 décrit dans Technical Information ME 151 e (977) juillet 1998 (MPM) de BASF Fine Chemicals.

8. Composition pharmaceutique selon la revendication 1 où le solubilisant non ionique a une valeur HLB ≥ 12.

9. Composition pharmaceutique selon la revendication 1 où le solubilisant non ionique est Solutol® HS 15 ou l'hydroxystéarate de polyéthylèneglycol 660.

10. Composition pharmaceutique selon l'une quelconque des revendications 1-9 où la concentration du solubilisant non ionique est dans un domaine de 0,001 % m/v à 30 % m/v, de préférence de 0,005 % m/v à 25 % m/v ; ou de préférence de 0,01 % m/v à 20 % m/v, de préférence à 15 %, de préférence encore à 10 % m/v, de manière particulièrement préférable à 8 % m/v ; de préférence encore de 0,05 % m/v à 6 % m/v, de préférence à 5 % m/v ; ou de préférence encore de 0,1 % m/v à 3 % m/v, ou de préférence encore de 0,2 % m/v à 2 % m/v ; et de manière particulièrement préférable de 0,3 % m/v à 1,5 % m/v ; ou de manière particulièrement préférable de 0,4 % m/v à 1 % m/v ; ou bien la concentration dudit solubilisant non ionique est de préférence d'environ 0,5 % m/v.

11. composition pharmaceutique selon la revendication 10, la composition comprenant de 0,0001 % m/v à 0,01 % m/v de séocalcitol et de 0,1 % m/v à 1,5 % m/v de Solutol® HS 15, de préférence de 0,0005 % m/v à 0,008 % m/v de séocalcitol et de 0,2 % à 1,0 % m/v de Solutol ® HS 15.

12. Composition pharmaceutique selon l'une quelconque des revendications 1-11 comprenant en outre moins de 1 % m/m, de préférence moins de 0,05 % m/m, de préférence encore environ 0,025 % m/m, de manière particulièrement préférable 0,01 % m/m d'un agent chélatant.

13. Composition pharmaceutique selon l'une quelconque des revendications 1-12 où le véhicule aqueux contient moins de 5 % m/m, de préférence moins de 4 % m/m, de préférence moins de 3 % m/m, de préférence encore moins de 2 % m/m, ou de manière particulièrement préférable moins de 1 % m/m d'un solvant organique.

14. Composition pharmaceutique selon la revendication 13 où le solvant organique est le polyéthylèneglycol ou le polypropylèneglycol.

15. Composition pharmaceutique selon l'une quelconque des revendications 1-14 où le pH de la composition est dans le domaine de 7,0 à 9,0, de préférence de 7,0 à 8,5, de préférence encore de 7,0 à 8,0, de manière particulièrement préférable de 7,0 à 7,8.

16. Composition pharmaceutique selon l'une quelconque des revendications 1-15 comprenant en outre un antioxydant qui a une solubilité dans l'eau qui est au moins 1,5 fois supérieure à sa solubilité dans le solubilisant non ionique.

17. Composition pharmaceutique selon la revendication 16, où l'antioxydant est choisi dans le groupe consistant en l'ascorbate de sodium, le bisulfite de sodium et le sulfite de sodium.

18. Composition pharmaceutique selon la revendication 17, où la concentration d'ascorbate de sodium dans la composition finale est de 0,2 % m/v à 3 % m/v, de préférence de 0,3 % m/v à 2,5 % m/v, de préférence encore de 0,4 % m/v à 2,0 % m/v, de manière particulièrement préférable d'environ 0,5 % m/v.

19. Utilisation d'une composition pharmaceutique selon l'une quelconque des revendications 1-18 pour la fabrication d'un médicament pour la prévention ou le traitement des troubles de la prolifération cellulaire, des troubles du métabolisme du calcium ou des maladies néoplasiques.

20. Utilisation selon la revendication 19 où lesdites maladies néoplasiques sont des hépatomes ; un cancer du pancréas, du sein, du colon ou de la prostate ; ou un trouble néoplasique hématologique.

21. Utilisation selon la revendication 19 où lesdits troubles du métabolisme du calcium sont l'ostéodystrophie rénale, l'hypoparathyroïdie, le rachitisme hypophosphatémique résistant à la vitamine D ou l'ostéomalacie.
